Europäisches Patentamt

European Patent Office

Office européen des brevets

(19)

(11) EP 0 896 049 B1

(12) **FASCICULE DE BREVET EUROPEEN**

(45) Date de publication et mention
de la délivrance du brevet:
**02.04.2003 Bulletin 2003/14**

(51) Int Cl.$^7$: **C10G 50/00**, C07C 2/76

(21) Numéro de dépôt: **98401844.0**

(22) Date de dépôt: **21.07.1998**

(54) **Procédé de conversion d'hydrocarbures légers gazeux en hydrocarbures supérieurs**

Verfahren zur Umsetzung von leichten gasförmigen Kohlenwasserstoffen in höhere Kohlenwasserstoffe

Process for the conversion of light gaseous hydrocarbons into higher hydrocarbons

(84) Etats contractants désignés:
**AT BE CH DE DK ES FI FR GB GR IE IT LI LU NL PT SE**

(30) Priorité: **04.08.1997 FR 9709964**

(43) Date de publication de la demande:
**10.02.1999 Bulletin 1999/06**

(73) Titulaire: **TotalfinaElf France**
**92800 Puteaux (FR)**

(72) Inventeurs:
• **Amariglio, Henri**
**54000 Nancy (FR)**

• **Pareja, Pierre**
**54600 Villers-les-Nancy (FR)**
• **Szabo, Georges**
**76290 Montvilliers (FR)**
• **Clemendot, Sylvain**
**14600 Honfleur (FR)**

(74) Mandataire: **Jolly, Jean-Pierre et al**
**Cabinet Jolly**
**54, rue de Clichy**
**75009 Paris (FR)**

(56) Documents cités:
**WO-A-92/01656        GB-A- 2 253 858**

**Description**

La présente invention concerne un procédé de conversion d'hydrocarbures légers gazeux, tels que ceux contenus par exemple dans le gaz naturel, en hydrocarbures supérieurs, de préférence liquides aux conditions normales de température et de pression.

L'abondance des champs de gaz naturel a amené les sociétés pétrolières à étudier la possibilité de convertir ce gaz sur le site même, afin de produire des hydrocarbures supérieurs liquéfiables à température ambiante, de façon à en faciliter notamment le stockage et le transport.

La voie la plus recherchée à ce jour nécessite le passage par un gaz de synthèse (mélange $H_2$ + CO), suivi de la réaction Fischer-Tropsch conduisant aux paraffines supérieures. Cette voie est cependant très coûteuse, car elle nécessite la mise en place d'équipements extrêmement complexes.

La recherche d'une voie plus directe a conduit à l'étude de méthodes de transformation du méthane en homologues supérieurs (d'où le nom d'homologation du méthane). Dans le cas de l'homologation non oxydante du méthane, on procède par déshydrogénation en alcanes supérieurs, selon la réaction :

$$nCH_4 \rightarrow C_n\ C_nH_{(2n+2)} + (n\text{-}1)\ H_2$$

Ce type de réaction de craquage thermique du méthane est toujours très défavorisé thermodynamiquement et, même en présence d'un catalyseur approprié, les rendements ne pourraient qu'être extrêmement faibles.

Afin de contourner cet obstacle thermodynamique, un nouveau procédé de nature catalytique a été envisagé. Il est décrit dans un article de la revue "Nature", 352, 1991, p.789-790, intitulé "Conversion of methane into higher hydrocarbons on platinum". Il s'agit d'un procédé séquentiel, comprenant deux étapes:

- Une première étape d'adsorption déshydrogénante, durant laquelle le catalyseur est mis en contact avec un courant de méthane à la pression atmosphérique et à une température de l'ordre de 250°C. Le méthane se chimisorbe de façon dissociative sur les sites métalliques du catalyseur supporté, avec une déshydrogénation progressive qui dépend du catalyseur et des conditions opératoires. Le schéma réactionnel correspondant est le suivant:

$$CH_4 \rightarrow CH_X + yH,$$

avec x+y = 4. Aux conditions opératoires, la majeure partie de l'hydrogène se désorbe et est entraînée par le méthane.
- Une seconde étape de désorption hydrogénante: l'ensemble catalyseur-fragments d'hydrocarbures adsorbés est mis en contact avec un courant d'hydrogène, à pression atmosphérique; les hydrocarbures supérieurs formés par hydrogénation sont désorbés et récupérés.

Cependant, les rendements de cette réaction d'homologation se sont avérés demeurer extrêmement faibles, bien que diverses propositions aient été faites afin de les améliorer:

- ainsi, la demande internationale de brevet WO 92/01656 préconise l'emploi d'un catalyseur constitué d'un métal de transition sur un support à base d'oxyde métallique réfractaire, à une température comprise entre 100 et 300°C : on fait passer alternativement sur le catalyseur un flux de méthane, puis un flux d'hydrogène ou, inversement, on fait circuler le catalyseur successivement dans le méthane, puis dans l'hydrogène, le catalyseur se présentant alors sous la forme d'un lit fluidisé circulant. Cependant, les rendements globaux de conversion par rapport au méthane ayant circulé sur le catalyseur ne dépassent pas 1,7%.
- selon le brevet GB 2253858, les températures des deux étapes diffèrent sensiblement; l'étape d'adsorption du méthane est effectuée avec du méthane dilué à 0,28% dans un gaz inerte (pression partielle de méthane de 0,0028.10$^5$Pa) et à température relativement élevée (entre 200 et 550°C), tandis que l'hydrogénation est réalisée à une température comprise de préférence entre la température ambiante et 150°C. A l'issue de cette deuxième étape, on récupère un mélange d'hydrocarbures légers ($C_1$ à $C_4$), dans lequel le taux de produits recherchés ($C_2$ à $C_4$) ne dépasse pas 20% ; en effet, environ 80% des hydrocarbures adsorbés redonnent du méthane lors de la désorption hydrogénante. Ce phénomène d'hydrogénolyse réduit donc d'autant l'intérêt du procédé, la sélectivité et les rendements restant médiocres.

Dans le cadre de ses travaux visant à améliorer ce type de procédé, la Demanderesse s'est particulièrement intéressée à l'étape au cours de laquelle s'effectue l'adsorption des hydrocarbures légers sur le catalyseur. Elle a constaté que cette étape d'adsorption est une véritable réaction chimique entre la surface métallique du catalyseur et les hydrocarbures légers constitutifs du gaz naturel: il y a formation d'espèces hydrocarbonées de type $CH_X$ (x<4), chimisorbées à la surface du catalyseur, ce phénomène s'accompagnant d'un dégagement d'hydrogène. Elle a émis l'hypothèse que l'hydrogène ainsi formé pouvait gêner l'adsorption des hydrocarbures à la surface du catalyseur et que l'on pouvait espérer améliorer cette première étape en éliminant, au coeur même de la réaction, l'hydrogène produit au fur et à mesure de sa formation.

La Demanderesse a par conséquent étudié la possibilité de réaliser l'étape d'adsorption en présence de composés susceptibles de fixer in situ l'hydrogène produit. Les résultats obtenus se sont révélés surprenants.

- d'une part, on constate une remarquable amélioration des quantités d'hydrocarbures légers homologuées en hydrocarbures supérieurs ; en effet, ces quantités sont mutipliées par un facteur de l'ordre de 1000.
- d'autre part, on observe une sélectivité inattendue de la conversion en faveur des hydrocarbures à 5 atomes de carbone et plus (notés par la suite $C_{5+}$). Or ces hydrocarbures, liquides aux conditions normales de température et de pression, présentent un intérêt supérieur par rapport aux hydrocarbures gazeux ($C_2$ à $C_4$), qui constituaient jusqu'alors les principaux produits obtenus en homologation du gaz naturel.

La fixation in situ de l'hydrogène peut être réalisée au moyen de tout composé susceptible, dans les conditions opératoires de la réaction d'adsorption, de réagir avec l'hydrogène pour former un hydrure. Compte tenu des applications industrielles de l'invention, ce composé doit être de plus facilement régénérable.

Dans cette optique, la Demanderesse a établi que les métaux et les composés métalliques peuvent être utilisés avec succès.

La présente invention a donc pour objet un procédé de conversion d'au moins un hydrocarbure léger, gazeux aux conditions normales de température et de pression, en hydrocarbures supérieurs, de préférence liquides aux conditions normales de température et de pression, ce procédé comportant au moins une étape d'adsorption du ou des hydrocarbures à convertir sur un catalyseur de conversion apte à former des espèces hydrocarbonées chimisorbées à la surface du catalyseur et étant caractérisé en ce que cette étape est réalisée en présence d'au moins un composé métallique différent du catalyseur et qui réagit avec l'hydrogène dégagé lors de l'adsorption du ou des hydrocarbures à convertir pour former un hydrure.

La fixation de l'hydrogène formé au cours de l'adsorption du ou des hydrocarbures à convertir est donc effectuée au moyen de composés métalliques susceptibles de réagir avec l'hydrogène pour former des hydrures. Ces composés seront appelés par la suite composés métalliques hydrurables.

Le choix d'un tel composé dépendra de différents paramètres :

- la stabilité thermique de l'hydrure aux conditions opératoires de la réaction d'adsorption des hydrocarbures légers à convertir ;
- l'enthalpie de formation de l'hydrure : la formation d'hydrure est en général une réaction exothermique, s'accompagnant d'un important dégagement de chaleur ;
- la capacité d'absorption de l'hydrogène par ledit composé métallique, qui doit être la plus élevée possible ;
- la facilité de régénération de ce composé métallique: il s'agit, dans des conditions opératoires appropriées, de décomposer l'hydrure en ce composé métallique hydrurable initial et en hydrogène ;
- la tenue mécanique de ce composé métallique, notamment dans des conditions d'utilisation cycliques (formation de l'hydrure, suivie de régénération du composé métallique hydrurable).

Ces composés métalliques hydrurables contiendront de préférence au moins deux métaux choisis parmi les métaux de transition et/ou les métaux du groupe des lanthanides et/ou les métaux du groupe des actinides et/ou les métaux du groupe des alcalins et des alcalino-terreux. Ces métaux pourront être utilisés purs, en alliage ou combinés à d'autres éléments de la Classification Périodique.

On utilisera avantageusement des métaux de transition, notamment des éléments des colonnes Ivb et/ou Vb et/ou VIII de la Classification Périodique des éléments, et en particulier des métaux choisis dans le groupe constitué par le zirconium, le titane, l'hafnium, le fer, le nickel, le cobalt, éventuellement combinés entre eux et/ou avec d'autres éléments. A titre d'exemple, on mentionnera $NiZr_2$, $FeHf_2$ et $LaNi_5$.

Le composé métallique hydrurable pourra être utilisé tel quel ou sous forme supportée, c'est à dire déposé sur tout type de support. Il pourra s'agir par exemple des supports usuellement employés pour les catalyseurs, tels que les oxydes de gallium, de titane, de thorium, de bore, les mélanges de ces oxydes, les silices, les alumines, les silice-alumines, les silicalites, les alumino-silicates, les magnésies, les zircones, les zéolithes, les charbons actifs.

Lors de l'étape d'adsorption, le composé métallique hydrurable pourra être mélangé ou non au catalyseur de conversion. De préférence, la quantité de ce composé sera optimisée de manière à fixer stoechiométriquement tout l'hydrogène formé lors de l'étape d'adsorption.

La régénération de ce composé métallique hydrurable consistera en une réaction de décomposition de l'hydrure; celle-ci pourra être effectuée par traitement thermique, en atmosphère inerte.

Comme indiqué ci-dessus et comme le montreront les exemples de mise oeuve exposés dans la suite de la présente description, le procédé selon la présente invention comporte divers avantages :

- en premier lieu, il permet d'effectuer la conversion des hydrocarbures légers constitutifs du gaz naturel en homologues supérieurs, avec une sélectivité inattendue en faveur des hydrocarbures liquides aux condition normales de température et de pression et une quantité de matière homologuée très fortement améliorée par rapport aux techniques jusqu'alors mises en oeuvre;
- par ailleurs, la présence des composés métalliques fixant l'hydrogène lors de l'étape d'adsorption permet d'améliorer l'efficacité de cette étape et d'opérer par conséquent à des températures relativement basses.. Ceci réduit les risques de formation, à la surface du catalyseur, d'espèces trop riches en carbone, non désorbables par la suite; à la longue, de telles espèces risquent d'amoindrir l'activité du catalyseur;
- de plus, compte tenu de son efficacité accrue, l'étape d'adsorption peut être réalisée avec des quantités de méthane beaucoup plus faibles que sans utilisation desdits composés métalliques hydrurables;
- enfin, la chaleur dégagée par la formation des hydrures pourra favoriser la dissociation des hydrocarbures gazeux sur le catalyseur qui, elle, est endothermique.

Dans le procédé selon la présente invention, le ou les hydrocarbures légers à convertir sont gazeux aux conditions normales de température et de pression. S'il s'agit d'un mélange, celui-ci est donc majoritairement constitué par des hydrocarbures renfermant de 1 1 à 4 atomes de carbone et, de préférence, majoritairement constitué de méthane et/ou d'éthane.

Comme catalyseur de conversion, on utilisera tout catalyseur usuellement mis en oeuvre dans les réactions d'homologation du méthane. De tels catalyseurs ont été largement décrits dans l'art antérieur.

Il s'agit en général de catalyseurs supportés métalliques, c'est à dire comprenant au moins un métal ou un alliage métallique déposé sur un support tel que, par exemple, un oxyde de gallium, de titane, de thorium, de bore, un mélange de ces oxydes, une silice, une alumine, une silice-alumine, une silicalite, un alumino-silicate, une magnésie, une zircone ou une zéolithe.

Les métaux ou alliages choisis pour ces catalyseurs pourront comprendre les métaux du groupe VIII de la Classification Périodique des éléments et, de préférence, ceux choisis parmi le platine, le cobalt, le nickel, le rhodium, le ruthénium, le palladium, le cuivre, ou un alliage de ces métaux.

Le nombre de sites actifs accessibles sur le support par unité de surface doit être élevé, de manière à pouvoir adsorber le maximum d'espèces hydrocarbures formées pendant l'étape d'adsorption. On retiendra donc des teneurs en métal élevées, comprises entre 1 et 50 % en poids par rapport au poids du support ainsi qu'une surface spécifique supérieure ou égale à 100 $m^2/g$ et un volume poreux supérieur ou égal à 0,2 $cm^3/g$.

Les catalyseurs utilisés dans la présente invention seront préparés selon les méthodes classiques connues, en déposant les précurseurs des métaux sur le support, puis en calcinant les solides ainsi obtenus. Puis une étape de réduction, utilisant de l'hydrogène, par exemple, sera pratiquée dans des conditions appropriées avant la mise en oeuvre du catalyseur.

Les conditions opératoires du procédé selon la présente invention seront choisies de manière à obtenir une conversion maximale de la charge en hydrocarbures liquides aux conditions normales de température et de pression.

L'étape d'adsorption du mélange gazeux sur le catalyseur sera conduite à une température de préférence supérieure ou égale à la température ambiante (25°C): la présence des composés métalliques hydrurables, en favorisant l'adsorption, permet d'opérer à des températures relativement basses. Cette température sera de préférence comprise entre 100°C et 500°C.

La pression, lors de l'étape d'adsorption, sera supérieure ou égale à $10^5$ Pa et, de préférence, comprise entre $10^5$ Pa et $60.10^5$ Pa.

La durée de cette première étape sera, de préférence, comprise entre 3 secondes et 10 minutes.

Ces divers paramètres seront optimisés de manière à bien maîtriser l'adsorption et à la rendre maximale, sans toutefois aller jusqu'à la formation d'espèces trop riches en carbone, non récupérables lors de la seconde étape. En d'autres termes, on cherchera à obtenir le maximum d'hydrocarbures légers homologués par rapport à la masse de catalyseur mise en oeuvre.

Avantageusement, le procédé selon la présente invention sera mis en oeuvre suivant deux étapes: la première étape d'adsorption du ou des hydrocarbures légers sur le catalyseur sera suivie d'une seconde étape permettant la récupération des hydrocarbures supérieurs formés sur la surface du catalyseur.

Cette seconde étape peut faire appel à tout moyen connu permettant de récupérer des hydrocarbures adsorbés sur une surface catalytique.

Elle pourra avantageusement consister en une désorption hydrogénante, par introduction d'hydrogène pur ou en mélange avec un ou plusieurs hydrocarbure(s) et/ou un ou plusieurs gaz inerte(s). Des alternatives à la désorption hydrogénante peuvent également être envisagées, comme par exemple la désorption par balayage de monoxyde de carbone ou d'un autre gaz approprié.

Dans le cas d'une désorption hydrogénante, celle-ci peut s'effectuer à la même température que l'étape de mise en contact avec le méthane ou à une température inférieure, mais au moins égale à 25°C. Celle-ci peut également être conduite selon un procédé polytherme, c'est-à-dire en faisant croître la température de manière progressive, de préférence de 25°C à 600°C, ou à l'intérieur de cet intervalle. Par ailleurs, la pression est avantageusement d'au moins $5.10^5$ Pa et, de préférence, comprise entre $10.10^5$ et $60.10^5$ Pa.

A l'issue de cette désorption hydrogénante, les hydrocarbures désorbés sont séparés de l'hydrogène. Les hydrocarbures liquides seront ensuite séparés des hydrocarbures gazeux par des méthodes usuelles telles que, par exemple, des méthodes de condensation, extraction, piégeage.

De manière avantageuse, le procédé selon la présente invention pourra être mis en oeuvre de façon continue et avec séparation, à l'issue de l'étape d'adsorption, du catalyseur de conversion et des hydrures métalliques. Ceux-ci peuvent alors être traités séparément avant d'être recyclés. Ainsi, le catalyseur pourra par exemple circuler entre un premier réacteur d'adsorption et un second réacteur de désorption hydrogénante, tandis que les composés métalliques hydrurables circuleront entre le réacteur d'adsorption et un réacteur de régénération, dans le même sens ou en sens contraire du sens de circulation du catalyseur.

Divers moyens peuvent être envisagés, afin de faciliter la séparation du catalyseur de conversion et des hydrures métalliques; on peut par exemple mettre à profit les propriétés magnétiques de certains composés métalliques hydrurables, ou la différence de densité des deux agents, ou encore utiliser des supports de natures différentes.

On peut également chercher à éviter le mélange du catalyseur de conversion et des composés métalliques hydrurables à l'intérieur du réacteur d'adsorption par diverses techniques connues de l'homme du métier, telles que, par exemple, l'utilisation de compartiments distincts séparés par des membranes perméables aux gaz.

Dans la même optique, on peut aussi mettre en oeuvre un réacteur d'adsorption dont les parois internes, à base de composé métallique hydrurable, permettraient l'évacuation de l'hydrogène formé au travers desdites parois.

De manière avantageuse, les hydrocarbures légers ainsi obtenus et se trouvant à l'état non liquide aux conditions normales de température et de pression pourront être recyclés partiellement ou totalement, en mélange avec le ou les hydrocarbures légers constituant la charge, ou séparément.

Les exemples qui suivent illustrent l'invention, sans toutefois la limiter.

Dans ces exemples, la charge que l'on souhaite convertir est constituée de méthane pur.

Le procédé de conversion est effectué en deux étapes, l'une d'adsorption du méthane sur le catalyseur, l'autre de désorption hydrogénante, au sein d'un réacteur contenant:

- 100 mg d'un catalyseur supporté bimétallique nickel/cuivre sur silice; ce catalyseur contient 16 % en poids de métaux et son rapport atomique, Cu/(Cu + Ni), est égal à 0,5; il présente une surface spécifique de 230 m²/g et un volume poreux de 0,6 cm³/g;
- une quantité donnée d'un composé métallique hydrurable, sous forme de poudre.

Pour la première étape (adsorption du méthane), on établit un vide primaire (à l'aide d'une pompe à palette) dans le réacteur, puis on met ce dernier en communication avec un réservoir contenant du méthane, dans des conditions de température et de pression données et pendant une durée donnée.

La deuxième étape (récupération des produits par désorption hydrogénante) est effectuée de la manière suivante: après trempe à la température ambiante et purge du réacteur par un courant de gaz inerte, on fait passer dans le réacteur un courant d'hydrogène avec un débit de 50 ml/mn, sous une pression donnée. Le débit gazeux est un débit volumique mesuré dans les conditions normales de température et de pression. La température est progressivement augmentée, de 25°C à 550°C.

La régénération du capteur d'hydrogène est effectuée par traitement thermique sous atmosphère inerte: le réacteur est porté à 600°C dans un courant d'argon (50 ml/mn) et maintenu à cette température pendant 1,5 à 2 heures.

Dans les exemples qui suivent, la quantité de méthane homologuée en hydrocarbures supérieurs (à 2 atomes de carbone et plus) sera exprimée en mg de méthane homologué par g de catalyseur. La sélectivité sera définie comme étant la quantité de méthane homologuée en hydrocarbures à 5 atomes de carbone et plus ($C_{5+}$) par rapport à la quantité totale de méthane homologuée en hydrocarbures supérieurs (à 2 atomes de carbone et plus). Ces $C_{5+}$ correspondent aux hydrocarbures liquides aux conditions normales de température et de pression.

Exemple 1

Dans cet exemple, comme composé métallique hydrurable, on utilise 1,4g de $NiZr_2$ sous forme de poudre, mélangée au catalyseur; les granulométries sont comparables et le mélange correspond approximativement à un volume de poudre pour un volume de catalyseur.

L'étape de désorption hydrogénante est réalisée sous une pression de $10^5$Pa.

La température de l'étape d'adsorption est fixée à 275°C, mais la pression ainsi que la durée de cette étape varient d'un essai à l'autre.

| Essai n° | Conditions d'adsorption | | Quantité de méthane homologuée | Sélectivité |
|---|---|---|---|---|
| | Pression ($10^5$Pa) | Durée (mn) | (en mg de $CH_4$/g de catalyseur) | ($C_{5+}/C_{2+}$ en %) |
| 1 | 1 | 0,5 | 1,06 | 70 |
| 2 | 1 | 2 | 1,16 | 74 |
| 3 | 1 | 5 | 1,5 | 79 |
| 4 | 4,8 | 0,5 | 1,37 | 71 |

Ces résultats montrent que le fait de réaliser l'étape d'adsorption en présence d'un composé métallique fixant l'hydrogène permet d'effectuer la conversion du méthane avec une excellente sélectivité en faveur des $C_{5+}$ et ce même à une faible pression (pression atmosphérique, comme c'est le cas pour les essais 1 à 3).

Les essais 1 à 3 montrent que la sélectivité ainsi que la quantité de méthane homologuée augmentent quelque peu avec la durée de l'étape d'adsorption (le gain porte essentiellement sur les $C_{8+}$).

Les essais 1 et 4 illustrent l'effet positif d'une pression plus forte lors de l'étape d'adsorption: on constate une amélioration des quantités de méthane homologuées, sans que cela nuise pour autant à la sélectivité.

Exemple 2

Dans cet exemple, comme composé métallique hydrurable, on utilise 1,5g de $FeHf_2$ sous forme de poudre, mélangée au catalyseur; les granulométries sont comparables.

La durée de l'étape d'adsorption est fixée à 2 mn, mais les conditions de température et de pression de cette étape varient d'un essai à l'autre.

L'étape de désorption hydrogénante est réalisée sous une pression de $10^5$Pa ou de $5.10^5$Pa selon l'essai.

| | Conditions d'adsorption | | | Quantité de méthane | |
|---|---|---|---|---|---|
| Essai n° | Pression $(10^5 Pa)$ | Température (°C) | Pression de désorption $(10^5 Pa)$ | homologuée (en mg de $CH_4$/g de catalyseur) | Sélectivité $(C_{5+}/C_{2+}$ en %) |
| 1 | 1 | 275 | 1 | 0,43 | 80 |
| 2 | 5 | 275 | 1 | 0,53 | 73,5 |
| 3 | 5 | 275 | 5 | 0,66 | 82,5 |
| 4 | 1 | 325 | 1 | 0,31 | 70 |
| 5 | 5 | 220 | 5 | 0,46 | 77 |

Ici encore, on notera les excellents résultats obtenus en présence du composé métallique fixant l'hydrogène au cours de l'étape d'adsorption; notamment, les sélectivités obtenues sont remarquables, puisqu'elles peuvent dépasser 80% en faveur des hydrocarbures liquides aux conditions normales de température et de pression (voir essai 3).

Les essais 1 1 à 3 illustrent l'influence des pressions mises en oeuvre lors des deux étapes; on retiendra notamment l'intérêt d'opérer à des pressions élevées (voir essai 3), tant du point de vue de la sélectivité que des quantités de méthane homologuées.

L'essai 4 souligne l'effet néfaste des températures d'adsorption élevées: on obtient de moins bons résultats, à cause des difficultés liées à la récupération d'espèces adsorbées trop riches en carbone. En revanche, grâce à la présence du composé métallique fixant l'hydrogène durant l'étape d'adsorption, il est possible de réaliser cette étape à des températures plus basses, tout en conservant de bons résultats; c'est ce que confirme l'essai 5.

Exemple 3

Dans cet exemple, les essais sont réalisés en présence de divers composés métalliques hydrurables; ces derniers sont utilisés sous forme de poudre, mélangée au catalyseur; les granulométries sont comparables.
L'étape d'adsorption est réalisée à une température de 275°C et sous une pression de $10^5 Pa$; sa durée est de 2 mn. L'étape de désorption hydrogénante est réalisée sous une pression de $10^5 Pa$.

| composé métallique hydrurable | Quantité de méthane homologuée (en mg de $CH_4$/g de catalyseur) | Sélectivité $(C_{5+}/C_{2+}$ en %) |
|---|---|---|
| $NiZr_2$ (1,4g) | 1,16 | 74 |
| $FeHf_2$ (1,5g) | 0,43 | 80 |
| $LaNi_5$ (1,7g) | 0,39 | 69 |

Cet exemple illustre la diversité des composés métalliques hydrurables pouvant être utilisés dans le procédé selon la présente invention.

Exemple 4

Cet exemple vise à comparer les résultats obtenus au moyen du procédé selon la présente invention avec les résultats que procure un procédé plus classique, tel que décrit dans l'art antérieur. En effet, l'expérience de conversion du méthane a été effectuée successivement en présence et en l'absence du composé métallique hydrurable, toutes les autres conditions opératoires étant identiques.

L'étape d'adsorption est effectuée à 275°C, sous -une pression de $10^5 Pa$ et pendant une durée de 5 mn. La désorption hydrogénante s'effectue sous une pression de $10^5 Pa$.

Pour l'essai en présence de composé métallique hydrurable, on emploie 1,4g de $NiZr_2$ sous forme de poudre, mélangée au catalyseur.

Les résultats obtenus apparaissent dans le tableau suivant

| Présence de NiZr$_2$ | Quantité de méthane homologuée (en mg de CH$_4$/g de catalyseur) |
|---|---|
| oui | 1,5 |
| non | 0,001 |

Ces résultats illustrent les progrès remarquables apportés par l'utilisation d'un composé métallique fixant l'hydrogène dans l'étape d'adsorption: la quantité de méthane homologuée est en effet multipliée par 1500.

## Revendications

1. Procédé de conversion d'au moins un hydrocarbure léger, gazeux aux conditions normales de température et de pression, en hydrocarbures supérieurs, de préférence liquides aux conditions normales de température et de pression, ce procédé comportant au moins une étape d'adsorption du ou des hydrocarbures à convertir sur un catalyseur de conversion apte à former des espèces hydrocarbonées chimisorbées à la surface du catalyseur et étant **caractérisé en ce que** cette étape est réalisée en présence d'au moins un composé métallique différent du catalyseur et qui réagit avec l'hydrogène dégagé lors de l'adsorption du ou des hydrocarbures à convertir pour former un hydrure.

2. Procédé selon la revendication 1, **caractérisé en ce que** ledit composé métallique contient au moins deux métaux choisis parmi les métaux de transition et/ou les métaux du groupe des lanthanides et/ou les métaux du groupe des actinides et/ou les métaux du groupe des alcalins et des alcalino-terreux, ces métaux pouvant être utilisés purs, en alliage ou combinés à d'autres éléments de la Classification Périodique.

3. Procédé selon la revendication 2, **caractérisé en ce que** ledit composé métallique contient au moins des éléments des colonnes Ivb et/ou Vb et/ou VIII de la Classification Périodique des éléments.

4. Procédé selon la revendication 3, **caractérisé en ce que** ledit composé métallique contient des métaux choisis dans le groupe constitué par le zirconium, le titane, l'hafnium, le fer, le nickel et le cobalt.

5. Procédé selon la revendication 4, **caractérisé en ce que** lesdits métaux sont utilisés en combinaison entre eux et/ou avec d'autres éléments.

6. Procédé selon l'une des revendications 1 à 5, **caractérisé en ce que** lesdits composés métalliques sont utilisés tels quels ou sous forme supportée, c'est à dire en dépôt sur tout type de support, dont les supports usuellement employés pour les catalyseurs.

7. Procédé selon l'une des revendications 1 à 6, **caractérisé en ce que** le catalyseur de conversion est un catalyseur supporté métallique, comprenant au moins un métal ou un alliage de métaux déposé(s) sur un support.

8. Procédé selon la revendication 7, **caractérisé en ce que** le support est choisi dans le groupe constitué par les oxydes de gallium, de titane, de thorium, de bore, les mélanges de ces oxydes, les silices, les alumines, les silice-alumines, les silicalites, les alumino-silicates, les magnésies, les zircones, les zéolithes.

9. Procédé selon l'une des revendications 7 et 8, **caractérisé en ce que** le catalyseur supporté métallique comprend au moins un métal choisi dans le groupe VIII de la Classification Périodique des éléments.

10. Procédé selon la revendication 9, **caractérisé en ce que** le catalyseur supporté métallique comprend au moins un métal choisi dans le groupe constitué par le platine, le cobalt, le nickel, le rhodium, le ruthénium, le palladium, le cuivre et les alliages de ces métaux.

11. Procédé selon l'une des revendications 7 à 10, **caractérisé en ce que** le catalyseur présente les caractéristiques suivantes : une teneur en métal comprise entre 1 et 50 % en poids par rapport au poids du support et/ou une surface spécifique supérieure ou égale à 100 m$^2$/g et/ou un volume poreux supérieur ou égal à 0,2 cm$^3$/g.

**12.** Procédé selon l'une des revendications précédentes, **caractérisé en ce que** les hydrocarbures légers à convertir forment un mélange majoritairement constitué par des hydrocarbures renfermant de 1 à 4 atomes de carbone.

**13.** Procédé selon la revendication 12, **caractérisé en ce que** les hydrocarbures légers à convertir forment un mélange majoritairement constitué de méthane et/ou d'éthane.

**14.** Procédé selon l'une des revendications précédentes, **caractérisé en ce que** l'étape d'adsorption est réalisée à une température supérieure ou égale à 25°C.

**15.** Procédé selon la revendication 14, **caractérisé en ce que** l'étape d'adsorption est réalisée à une température comprise entre 100°C et 500°C.

**16.** Procédé selon l'une des revendications précédentes, **caractérisé en ce que** l'étape d'adsorption est réalisée à une pression supérieure ou égale à 10$^5$ Pa.

**17.** Procédé selon la revendication 16, **caractérisé en ce que** l'étape d'adsorption est réalisée à une pression comprise entre 10$^5$ Pa et 60.10$^5$ Pa.

**18.** Procédé selon l'une des revendications précédentes, **caractérisé en ce que** l'étape d'adsorption s'effectue pendant une durée comprise entre 3 secondes et 10 minutes.

**19.** Procédé selon l'une des revendications précédentes, **caractérisé en ce que** l'étape d'adsorption des hydrocarbures légers sur le catalyseur est suivie d'une seconde étape de récupération des hydrocarbures supérieurs formés sur la surface du catalyseur.

**20.** Procédé selon la revendication 19, **caractérisé en ce que** la seconde étape de récupération des hydrocarbures supérieurs est une désorption hydrogénante, effectuée au moyen d'hydrogène pur, ou en mélange avec un ou plusieurs hydrocarbures(s) et/ou un ou plusieurs gaz inerte(s).

**21.** Procédé selon la revendication 20, **caractérisé en ce que** la désorption hydrogénante est réalisée dans les conditions suivantes :

- une pression d'au moins 5.10$^5$ Pa ;
- une température fixe, inférieure ou égale à celle de l'étape d'adsorption ou une température augmentant progressivement à l'intérieur de l'intervalle compris entre 25°C et 600°C.

**22.** Procédé selon la revendication 21, **caractérisé en ce que** la pression est comprise entre 10.10$^5$ et 60.10$^5$ Pa.

**23.** Procédé selon l'une des revendications précédentes, **caractérisé en ce que**, à l'issue de l'étape d'adsorption, le catalyseur et les hydrures métalliques sont séparés, avant d'être traités et recyclés.

**24.** Procédé selon l'une des revendications précédentes, **caractérisé en ce que** les hydrocarbures produits et se trouvant à l'état non liquide aux conditions normales de température et de pression, sont recyclés partiellement ou totalement, en mélange avec le ou les hydrocarbures légers constituant la charge, ou séparément.

**Claims**

**1.** A process for converting at least one light hydrocarbon, gaseous under normal temperature and pressure conditions, into higher hydrocarbons, preferably liquid under normal temperature and pressure conditions, said process comprising at least one stage involving adsorption of the hydrocarbon(s) to be converted on a conversion catalyst capable of forming hydrocarbon species chemisorbed on the surface of the catalyst and being **characterised in that** this stage is performed in the presence of at least one metal compound different from the catalyst and which reacts with the hydrogen released during adsorption of the hydrocarbon(s) to be converted to form a hydride.

**2.** A process according to claim 1, **characterised in that** said metal compound contains at least two metals selected

9

from among the transition metals and/or the metals from the lanthanide group and/or the metals from the actinide group and/or the metals from the alkali and alkaline-earth group, said metals being capable of being used in the pure state, as alloys or combined with other elements from the Periodic Table.

3. A process according to claim 2, **characterised in that** said metal compound contains at least elements from the columns IVb and/or Vb and/or VIII of the Periodic Table of elements.

4. A process according to claim 3, **characterised in that** said metal compound contains metals selected from the group consisting of zirconium, titanium, hafnium, iron, nickel and cobalt.

5. A process according to claim 4, **characterised in that** said metals are used in combinations of one another and/ or with other elements.

6. A process according to one of claims 1 to 5, **characterised in that** said metal compounds are used as they are or in supported form, that is to say deposited on any type of support, including the supports conventionally used for catalysts.

7. A process according to one of claims 1 to 6, **characterised in that** the conversion catalyst is a supported metal catalyst, comprising at least one metal or one metal alloy deposited on a support.

8. A process according to claim 7, **characterised in that** the support is selected from the group consisting of gallium, titanium, thorium and boron oxides, mixtures of these oxides, silicas, aluminas, silica-aluminas, silicalites, aluminosilicates, magnesias, zirconias and zeolites.

9. A process according to one of claims 7 and 8, **characterised in that** the supported metal catalyst comprises at least one metal selected from group VIII of the Periodic Table of Elements.

10. A process according to claim 9, **characterised in that** the supported metal catalyst comprises at least one metal selected from the group consisting of platinum, cobalt, nickel, rhodium, ruthenium, palladium, copper and alloys of these metals.

11. A process according to one of claims 7 and 10, **characterised in that** the catalyst has the following characteristics: a metal content of between 1 and 50% by weight relative to the weight of the support and/or a specific surface area greater than or equal to 100 $m^2$/g and/or a pore volume greater than or equal to 0.2 $cm/g^3$.

12. A process according to one of the preceding claims, **characterised in that** the light hydrocarbons to be converted form a mixture consisting for the most part of hydrocarbons containing from 1 to 4 carbon atoms.

13. A process according to claim 12, **characterised in that** the light hydrocarbons to be converted form a mixture consisting for the most part of methane and/or ethane.

14. A process according to one of the preceding claims, **characterised in that** the adsorption stage is performed at a temperature greater than or equal to 25°C.

15. A process according to claim 14, **characterised in that** the adsorption stage is performed at a temperature of between 100°C and 500°C.

16. A process according to one of the preceding claims, **characterised in that** the adsorption stage is performed at a pressure greater than or equal to $10^5$ Pa.

17. A process according to claim 16, **characterised in that** the adsorption stage is performed at a pressure of between $10^5$ Pa and $60.10^5$ Pa.

18. A process according to one of the preceding claims, **characterised in that** the adsorption stage is performed for a period of between 3 seconds and 10 minutes.

19. A process according to one of the preceding claims, **characterised in that** the stage involving adsorption of the light hydrocarbons on the catalyst is followed by a second stage involving recovery of the higher hydrocarbons formed on the surface of the catalyst.

20. A process according to claim 19, **characterised in that** the second stage involving recovery of the higher hydrocarbons is hydrogenating desorption, performed by means of pure hydrogen or of hydrogen in a mixture with one or more hydrocarbons and/or one or more inert gases.

21. A process according to claim 20, **characterised in that** the hydrogenating desorption is performed under the following conditions:

- a pressure of at least $5.10^5$ Pa;
- a fixed temperature, lower than or equal to that of the adsorption stage, or a temperature increasing progressively within the range between 25°C and 600°C.

22. A process according to claim 21, **characterised in that** the pressure is between $10.10^5$ and $60.10^5$ Pa.

23. A process according to one of the preceding claims, **characterised in that**, at the end of the adsorption stage, the catalyst and the metal hydrides are separated, before being treated and recycled.

24. A process according to one of the preceding claims, **characterised in that** the hydrocarbons produced and in a non-liquid state under normal temperature and pressure conditions are partially or totally recycled, in a mixture with the light hydrocarbon(s) constituting the feedstock or separately.

**Patentansprüche**

1. Verfahren zur Umwandlung mindestens eines leichten, bei normalen Temperatur- und Druckbedingungen gasförmigen Kohlenwasserstoffs, in höhere Kohlenwasserstoffe, die unter normalen Temperatur- und Druckbedingungen vorzugsweise flüssig sind, wobei dieses Verfahren mindestens eine Stufe der Adsorption des oder der umzuwandelnden Kohlenwasserstoffe auf einem Umwandlungskatalysator umfasst, der in der Lage ist, chemisorbierte Kohlenwasserstoff-Spezies auf seiner Oberfläche des Katalysators zu bilden und das **dadurch gekennzeichnet ist, dass** diese Stufe in Gegenwart mindestens einer Metallverbindung durchgeführt wird, die sich vom Katalysator unterscheidet und die mit dem bei der Adsorption des oder der umzuwandelnden Kohlenwasserstoffe abgegebenen Wasserstoff reagiert, um sich in ein Hydrid umzuwandeln.

2. Verfahren nach Anspruch 1, **dadurch gekennzeichnet, dass** die Metallverbindung mindestens zwei Metalle, ausgewählt aus den Übergangsmetallen und/oder den Metallen aus der Gruppe der Lanthaniden und/oder den Metallen aus der Gruppe der Aktiniden und/oder den Metallen der Gruppe der Alkalien und der Erdalkalien, enthält, wobei diese Metalle in reiner Form, als Legierung oder in Kombination mit anderen Elementen des Periodensystems verwendet werden können.

3. Verfahren nach Anspruch 2, **dadurch gekennzeichnet, dass** die Metallverbindung mindestens die Elemente der Gruppen IVb und/oder Vb und/oder VIII des Periodensystems der Elemente enthält.

4. Verfahren nach Anspruch 3, **dadurch gekennzeichnet, dass** die Metallverbindung Metalle enthält, die aus der Gruppe, bestehend aus Zirkon, Titan, Hafnium, Eisen, Nickel und Kobalt ausgewählt sind.

5. Verfahren nach Anspruch 4, **dadurch gekennzeichnet, dass** die Metalle in Kombinationen miteinander und/oder mit anderen Elementen verwendet werden.

6. Verfahren nach einem der Ansprüche 1 bis 5, **dadurch gekennzeichnet, dass** die Metallverbindungen als solche oder auf Trägern, d.h. abgeschieden auf jeder Art von Trägern verwendet werden, deren Träger üblicherweise für Katalysatoren verwendet werden.

7. Verfahren nach einem der Ansprüche 1 bis 6, **dadurch gekennzeichnet, dass** der Umwandlungskatalysator ein metallischer Trägerkatalysator ist, der mindestens ein Metall oder eine Legierung von Metallen enthält, das (die) auf einem Träger abgeschieden ist (sind).

8. Verfahren nach Anspruch 7, **dadurch gekennzeichnet, dass** der Träger aus der Gruppe, bestehend aus Oxiden des Galliums, des Titans, des Thoriums, des Bors, aus Mischungen dieser Oxide, aus Siliciumoxiden, aus Aluminiumoxiden, aus Siliciumoxid-Aluminium-oxiden, aus Silicaliten, aus Alumino-Silicaten, aus Magnesiumoxiden, aus Zirkonoxiden, aus Zeolithen ausgewählt ist.

9. Verfahren nach einem der Ansprüche 7 und 8, **dadurch gekennzeichnet, dass** der metallische Trägerkatalysator mindestens ein Metall, ausgewählt aus der Gruppe VIII des Periodensystems der Elemente, enthält.

10. Verfahren nach Anspruch 9, **dadurch gekennzeichnet, dass** der metallische Trägerkatalysator mindestens ein Metall, ausgewählt aus der Gruppe, bestehend aus Platin, Kobalt, Nickel, Rhodium, Ruthenium, Palladium, Kupfer und den Legierungen dieser Metalle, enthält.

11. Verfahren nach einem der Ansprüche 7 bis 10, **dadurch gekennzeichnet, dass** der Katalysator folgende Eigenschaften aufweist: einen Metallgehalt zwischen 1 und 50 Gew.-%, bezogen auf das Gewicht des Trägers, und/oder eine spezifische Oberfläche von $\geq$ 100 m$^2$/g und/oder ein Porenvolumen von $\geq$ 0,2 cm$^3$/g.

12. Verfahren nach einem der vorhergehenden Ansprüche, **dadurch gekennzeichnet, dass** die umzuwandelnden leichten Kohlenwasserstoffe ein Gemisch darstellen, dass hauptsächlich aus Kohlenwasserstoffen mit 1 bis 4 Kohlenstoffatomen zusammengesetzt ist.

13. Verfahren nach Anspruch 12, **dadurch gekennzeichnet, dass** die umzusetzenden leichten Kohlenwasserstoffe ein hauptsächlich aus Methan und/oder Ethan zusammengesetztes Gemisch bilden.

14. Verfahren nach einem der vorhergehenden Ansprüche, **dadurch gekennzeichnet, dass** die Stufe der Adsorption bei einer Temperatur von mehr als oder gleich 25°C durchgeführt wird.

15. Verfahren nach Anspruch 14, **dadurch gekennzeichnet, dass** die Stufe der Adsorption bei einer Temperatur zwischen 100 und 500°C durchgeführt wird.

16. Verfahren nach einem der vorhergehenden Ansprüche, **dadurch gekennzeichnet, dass** die Stufe der Adsorption bei einem Druck von mehr als oder gleich 10$^5$ Pa durchgeführt wird.

17. Verfahren nach Anspruch 16, **dadurch gekennzeichnet, dass** die Stufe der Adsorption bei einem Druck zwischen 10$^5$ Pa und 60 x 10$^5$ Pa durchgeführt wird.

18. Verfahren nach einem der vorhergehenden Ansprüche, **dadurch gekennzeichnet, dass** die Stufe der Adsorption innerhalb eines Zeitraums von 3 Sekunden bis 10 Minuten durchgeführt wird.

19. Verfahren nach einem der vorhergehenden Ansprüche, **dadurch gekennzeichnet, dass** auf die Stufe der Adsorption der leichten Kohlenwasserstoffe auf dem Katalysator eine zweite Stufe der Wiedergewinnung der auf der Oberfläche des Katalysators gebildeten höheren Kohlenwasserstoffe folgt.

20. Verfahren nach Anspruch 19, **dadurch gekennzeichnet, dass** die zweite Stufe der Wiedergewinnung der höheren Kohlenwasserstoffe eine hydrierende Desorption darstellt, die mit Hilfe von reinem Wasserstoff oder im Gemisch mit einem oder mehreren Kohlenwasserstoff(en) und/oder einem oder mehreren Inertgas(en) durchgeführt wird.

21. Verfahren nach Anspruch 20, **dadurch gekennzeichnet, dass** die hydrierende Desorption unter folgenden Bedingungen durchgeführt wird:

- bei einem Druck von mindestens 5 x 10$^5$ Pa;
- bei einer festen Temperatur, die gleich oder niedriger als die Temperatur auf der Stufe der Adsorption ist, oder bei einer sich innerhalb des Intervalls zwischen 25°C und 600°C progressiv erhöhenden Temperatur.

22. Verfahren nach Anspruch 21, **dadurch gekennzeichnet, dass** der Druck zwischen 10 x 10$^5$ und 60 x 10$^5$ Pa liegt.

23. Verfahren nach einem der vorhergehenden Ansprüche, **dadurch gekennzeichnet, dass** am Ende der Stufe der Adsorption der Katalysator und die Metallhydride abgetrennt werden, bevor sie behandelt und zurückgeleitet werden.

**24.** Verfahren nach einem der vorhergehenden Ansprüche, **dadurch gekennzeichnet, dass** die Kohlenwasserstoffprodukte, die sich bei normalen Temperatur- und Druckbedingungen nicht im flüssigen Zustand befinden, im Gemisch mit oder getrennt von dem oder den leichten Kohlenwasserstoff(en), die die Beschickung darstellen, teilweise oder ganz zurückgeleitet werden.